# EUROPEAN PATENT SPECIFICATION

(11) **EP 1 719 527 B1**
(45) Date of publication and mention of the grant of the patent: **04.05.2016**
(21) Application number: 05709501.0
(22) Date of filing: 31.01.2005
(51) Int. Cl.: A61K 47/04, A61K 31/282, A61K 31/407, A61K 31/475, A61K 31/505, A61K 31/513, A61K 31/675, A61K 31/704, A61K 31/7048, A61K 31/706, A61K 38/21, A61P 35/00

(54) **ANTITUMOR AGENTS**
ANTITUMORALE MITTEL
AGENTS ANTITUMORAUX

(30) Priority: 09.02.2004 JP 2004032184; 05.08.2004 JP 2004229055
(43) Date of publication of application: 08.11.2006
(73) Proprietor: KABUSHIKI KAISHA SANGI, Tokyo 104-0045 (JP)
(72) Inventor: SAKUMA, Shuji, c/o Kabushiki Kaisha Sangi,, Tokyo 1040045 (JP); ATSUMI, Kiminori, c/o Kabushiki Kaisha Sangi,, Tokyo 1040045 (JP); KIKUKAWA, Keiichiro, c/o Kabushiki Kaisha Sangi,, Tokyo 1040045 (JP)
(74) Representative: Vossius & Partner Patentanwälte Rechtsanwälte mbB
(86) International application number: PCT/JP2005/001338
(87) International publication number: WO 2005/074991

(56) References cited:
- EP-A1- 1 295 607
- WO-A1-03/030943
- WO-A2-01/28587
- WO-A2-02/41844
- JP-A- 2 200 628
- JP-A- 4 074 125
- JP-A- 4 112 832
- JP-A- 6 329 557
- JP-B2- 1 051 266
- AOKI H. ET AL: 'In vitro interaction of carcinostatic substances adsorbed on hydroxyapatite microcrystals with cells derived from cancers.' TRANSACTIONS OF THE MATERIAL RESEARCH SOCIETY OF JAPAN. vol. 15A, 1994, pages 3 - 9, XP002989810
- LIU Z.S. ET AL: 'Effects of hydroxyapatite nanoparticles on proliferation and apoptosis of human hepatoma BEL-7402 cells.' WORLD J. GASTROENTEROL. vol. 9, no. 9, 2003, pages 1968 - 1971, XP002989886

## Description

### Technical Field

The present invention relates to antitumor agents with reduced toxicity that can be used for oral administration.

### Background Art

In chemotherapy, various antitumor agents have been developed, for example, alkylating agents that destruct cancer cell DNA or inhibit its replication by introducing an alkyl group to various tumors such as stomach cancer, esophagus cancer, liver cancer, colon cancer, rectal cancer, pancreatic cancer, lung cancer, thyroid gland cancer, breast cancer, ovarian cancer, cervical cancer, endometrial cancer, ovarian cancer, tongue cancer, lip cancer, pharyngeal cancer, laryngeal cancer, mouth cancer, lung cancer, skin cancer, malignant melanoma, rhabdomyosarcoma, ureteral tumor, bladder cancer, prostate cancer, testicular tumor, malignant lymphoma, leukemia, myeloma, bone tumor, neural tumor, and glioma; antimetabolites that suppress cancer cell growth by disturbing cancer cell function through inhibition of cancer cell metabolism; antitumor antibiotics of the antibiotics obtained from naturally occurring microorganisms, that have carcinostatic effects such as destroying cancer cell membrane, degrading DNA and inhibiting DNA synthesis; plant preparations that halt cell division and impart cell damage by plant alkaloids obtained from plants; hormone preparations that show antitumor effects by binding to a hormone-binding site of cancer cells, a hormonal agent that has an opposite effect to the hormone, or a hormone antagonist; immunoactivators for activating the immune system, or immunotherapeutic agents, such as cytokines, for regulating and/or enhancing immune responses against cancer; platinum preparations that inhibit division of cancer cells by binding to their DNA; and other antitumor agents that are not classified as above, such as kinase inhibitors, histamine A derivatives, aminopeptidases of Streptomyces, mannitol derivatives having effects similar to those of alkylating agents and antimetabolites, enzyme preparations that place asparagine auxotrophic tumor cells in a nutritionally-deficient condition by decomposing blood L-asparagine, bisdioxopiperazine derivatives, and aceglatone which is an agent that suppresses bladder tumor recurrence. Further, various therapeutic methods have been developed, for example, surgical therapy, radiation therapy, proton beam therapy, immunotherapy, lymphocyte therapy, gene therapy, and thermotherapy, and have led to improved therapeutic effects when used in combination.

On the other hand, in cancer therapy by antitumor agent administration, antitumor agents have various effects on normal cells and side effects appear; and none of them selectively acts on cancer cells. Accordingly, in the real world, the drugs are not used to their full original effect because of the restricted use of antitumor agents and the inevitable discontinuation of drug administration due to side effects that are intolerable to patients.

Despite useful effects of many compounds as antitumor agents, their development was halted because of the side effects and toxicity.

It is desirable to develop auxiliary agents that have the effect of reducing side effects and toxicity of antitumor agents, as well as antitumor agents containing such a substance.

Hydroxyapatite, usually represented by the stoichiometric composition of Ca₁₀(PO₄) ₆(OH)₂, is the principal inorganic component of bones and teeth. Since hydroxyapatite has high biocompatibility and functions to adsorb proteins and such, it is manufactured into, for example, artificial bones, bone-filling agents, and dentifrice, or devised to be an auxiliary component of pharmaceutical agents.

A method for suppressing tumor growth by injecting into an artery that leads to the tumor site, hydroxyapatite of an average particle size of 10 to 1000 µm attached with a carcinostatic agent, and retaining it as a microembolus to cut off the nutritional supply to tumor and maintain a high concentration of the carcinostatic agent at the tumor site for a long time (Japanese Patent Application Kokoku Publication No. (JP-B) H1-51266 (examined, approved Japanese patent application published for opposition)); a method for sustaining the release of a pharmaceutical agent by implanting into the body hydroxyapatite (100 to 500 µm) filled with an antitumor agent (Japanese Patent Application Kokai Publication No. (JP-A) H2-200628 (unexamined, published Japanese patent application)); a method for accelerating or delaying the effect of a pharmaceutical agent by adding a pharmaceutical agent to calcium phosphate microcrystals and administering them to blood vessels, or a method for selectively adsorbing calcium phosphate microcrystals with, for example, various cells such as cancer cells, or viruses such as the AIDS virus, ATL virus, and hepatitis virus, to control their differentiation and growth and at the same time, allow the pharmaceutical agent to take effect (JP-A H5-255095); a method of using thermochemotherapy by implanting hydroxyapatite attached with an antitumor agent (1,250 µm to 1,500 µm) into a tumor site, followed by heating ("Cancer and chemotherapy" 19(10): 1644-1647, 1992); and a method for sustaining the release of carboplatin, by using porous hydroxyapatite with an average particle size of 36.1 µm and surface area of 2.5 m²/g as a sustained-release preparation to produce and adjust sustained-release carboplatin, and then administering it to the abdominal cavity or mediastinal space of the thoracic cavity ("Cancer and chemotherapy" 26(12): 1791-1793, 1999) are disclosed.
[Patent Document 1] JP-B H1-51266
[Patent Document 2] JP-A H2-200628
[Patent Document 3] JP-A H5-255095
[Non-patent Document 1] "Cancer and chemotherapy" 19(10): 1644-1647, 1992
[Non-patent Document 2] "Cancer and chemotherapy" 26(12): 1791-1793, 1999

EP1295607 describes a pharmaceutical composition for the treatment of a tumor, comprising a compound having a Rho kinase inhibitory activity, an antitumor agent and a pharmaceutically acceptable carrier. The antitumor agent can be e.g. etoposide, nedaplatin, goserelin acetate, melphalan, thiotepa or cytarabine ocfosfate. The effect of the antitumor agent is reinforced by means of the Rho kinase inhibitor.

### Disclosure of the Invention

The present disclosure aims to provide methods for reducing toxicity of antitumor agents without reducing their effect. Antitumor agents with reduced toxicity, and antitumor agents for oral administration are provided by the present invention. As disclosed herein, the antitumor agent may also be administered by intravascular, intraperitoneal, intramuscular, and subcutaneous administration via injection, infusion, and such.

The present inventors have discovered that the toxicity of antitumor agents may be reduced while maintaining the antitumor effects of antitumor agents, by supporting antitumor agents with hydroxyapatite microparticles and administering them by oral administration, and thus completed the present invention.
In particular, the present invention provides the following:
1. An antitumor agent comprising an antitumor component blended with a hydroxyapatite, wherein the antitumor component is etoposide, interferon-β, nedaplatin, nimustine hydrochloride, carboquone, melphalan, ifosfamide, thiotepa, vinorelbine tartrate, tegafur, goserelin acetate, sobuzoxane, tretinoin, estramustine sodium phosphate, toremifene citrate, hydroxy carbamide, cytarabine ocfosfate, doxifluridine, or gefinitib, wherein hydroxyapatite has a maximum particle size of 5 µm or less, and wherein the antitumor agent is for oral administration.
2. The antitumor agent of item 1, wherein the antitumor component is interferon-β, nedaplatin, nimustine hydrochloride, carboquone, vinorelbine tartrate, tegafur, sobuzoxane, tretinoin, estramustine sodium phosphate, toremifene citrate, hydroxy carbamide, cytarabine ocfosfate, doxifluridine, or gefinitib.
3. The antitumor agent of item 1, wherein the antitumor component is interferon-β, nedaplatin, carboquone, vinorelbine tartrate, tegafur, or tretinoin.
The antitumor agent of any one of items 1 to 3, comprising hydroxyapatite with a maximum particle size of 1 µm or less.
The antitumor agent of any one of items 1 to 4, comprising hydroxyapatite with a maximum particle size of 0.5 µm or less, wherein the antitumor agent is for oral adminstration.
The antitumor agent of any one of items 1 to 5, comprising hydroxyapatite with a maximum particle size of 0.1 µm or less.
The antitumor agent of any one of items 1 to 6, wherein the amount of hydroxyapatite blended is 0.1 to 1000% of the antitumor component.
The antitumor agent of any one of items 1 to 7, wherein the mixture of antitumor component and hydroxyapatite is pulverised.
An antitumor agent of any one of items 1 to 8 for use in reducing toxicity of an antitumor component of item 1, wherein the antitumor agent is to be administered by oral administration.

As described above, because of their biocompatibility and adsorption of proteins and such, hydroxyapatites are manufactured into artificial bones, bone-filling agents, and dentifrice. Further, hydroxyapatite particles adsorbed with an antitumor agent are used as a microembolus for cutting off the nutritional supply to tumor by injecting them into an artery, and maintaining a high concentration of carcinostatic agent at the tumor site for a long time; as a sustained-release agent to be implanted into the body for sustained release of antitumor agents; and as a material in thermochemotherapy to be implanted into tumor sites followed by heating. Methods that accelerate or delay the effects of pharmaceutical agents by intravascular administration of hydroxyapatite microparticles adsorbed with a pharmaceutical agent to control the differentiation and growth of cancer cells or viruses while allowing the pharmaceutical agent to take effect, and methods that administer porous hydroxyapatite particles treated with an antitumor agent to a cavity for sustained release of the antitumor agent are disclosed. However, their use as an embolus requires considerable skills. Al1 other methods aim for the sustained release of antitumor agents, which fail to bring out the full antitumor effect of each antitumor agent.

The present invention reveals that by mixing and adsorbing hydroxyapatites with various antitumor agents, and then by intravascularly administering an appropriate amount of various antitumor agents, toxicity of the antitumor agents may be reduced while maintaining their antitumor effects.

The hydroxyapatites used in the present invention are usually represented by the stoichiometric composition of Ca₁₀(PO₄) ₆(OH)₂. However, even in non-stoichiometric cases where the Ca/P molar ratio is not 1.67, they show hydroxyapatite properties and have the characteristic of forming apatite structures.

The present invention can use hydroxyapatites with either a stoichiometric composition or non-stoichiometric composition, and can use those with a Ca/P molar ratio of 1.4 to 1.8.

The Ca/P molar ratio of hydroxyapatite is regulated by controlling the mixing ratio of ingredient salts and the synthetic conditions. For example, in wet synthesis, the Ca/P molar ratio is high when the aqueous solution is adjusted to basic using aqueous ammonia and such at the time of synthesis, whereas the Ca/P molar ratio can be lowered by using a dilute acid and adjusting the aqueous solution to a neutral or weak acid.

The above-mentioned hydroxyapatites used in the present invention may be either crystalline or low-crystalline; however, low-crystalline or non-crystalline hydroxyapatites may also be used.

The term "low-crystalline" as used herein refers to crystalline powders with a broader X-ray diffraction peak as compared with high-crystalline powders.

The term "non-crystalline" refers to powders comprising microparticles that display a broad halo in their X-ray diffraction pattern, but for which a diffraction pattern that shows crystal characteristics cannot be obtained. Herein below, low-crystalline hydroxyapatites and non-crystalline hydroxyapatites are respectively referred to as "low-crystalline apatites" and "non-crystalline apatites".

The low-crystalline apatites or non-crystalline apatites used in the present invention comprise, for example, apatites synthesized by wet synthesis as described above and then freeze-dried or dried at a temperature of 100°C or less, or those baked at a temperature of about 300°C or less.

Low-crystalline apatites or non-crystalline apatites comprise particles with a smaller particle size as compared with high-crystalline hydroxyapatites (herein below referred to as "crystalline apatites").

Hydroxyapatite particles used in the present invention preferably have a maximum particle size of 1.0 µm or less. Since the specific surface area increases as the average particle size decreases, which heightens the capacity for adsorption of pharmaceutical agents, those with a maximum particle size of 0.1 µm or less are more preferable. Due to similar reasons, lower limits of the average particle size of particles may also be used.
In oral administration of antitumor agents that work by gastric absorption or antitumor agents that work by intestinal absorption, if a gastric acid-insolubilization treatment by encapsulation, coating, and such is not performed to prevent the hydroxyapatites from being decomposed by gastric acid, the maximum particle size is preferably 5 µm or less, and more preferably 0.5 µm or less.

The method for adjusting the maximum particle size of hydroxyapatite particles to be 5 µm or less, 1.0 µm or less, 0.5 µm or less, and 0.1 µm or less can be carried out by pulverization. Hydroxyapatites may be mixed with various antitumor agents after pulverization and then used; however, to reduce the toxicity of various antitumor agents, pulverization may also be performed after supporting the antitumor agents with hydroxyapatites.

Powder and solid antitumor agents may be used by mixing them intact with hydroxyapatite. Pulverization is performed after dissolving a powder or solid antitumor agent and supporting it with hydroxyapatite. Other than antitumor agents that are dissolved in solvents which can be safely used for injection and/or infusion, such as distilled water or physiological saline, solvents may be removed to reduce toxicity of various antitumor agents by methods such as drying the thus obtained antitumor solutions before use.

The blending quantity of hydroxyapatite used to support an antitumor agent differs depending on the antitumor agent used, which makes generalization difficult, but is preferably 0.1 to 1000% of the antitumor agent, and suitably 0.1 to 500% in consideration of the antitumor agent dosage, and 1 to 200% further in view of the toxicity-reducing effect and antitumor agent dosage.

The Examples of the present invention will be described below; however, the following Examples describe examples that use LD50 measurement as the toxicity test.

### Effects of the Invention

The present invention provides antitumor agents that make the best use of their original antitumor effect, because the toxicity of various side effect-carrying antitumor agents is reduced.

### Brief Description of the Drawings

Fig. 1 is a graph showing cell growth inhibition curves.
Fig. 2 is a graph showing cell growth inhibition curves

### Best Mode for Currying Out the Invention

Herein below, the present invention will be described in detail with reference to examples, that use an antitumor component as defined in independent claim 1, and reference-examples that use further antitumor components.

### Examples and reference-examples

### [Preparation of various hydroxyapatite-supplemented antitumor agent solutions]

Various hydroxyapatite-suspended antitumor solutions were produced by adding a given amount of each of the various antitumor agents to 2% hydroxyapatite-supplemented distilled water, followed by stirring. To accelerate the reaction, antitumor agents, other than cyclophosphamide and interferon β, were stirred in darkness in a 50°C high-temperature bath and under reduced pressure to avoid any influence of air exposure.

Cyclophosphamide and interferon β were stirred at 4°C by a procedure similar to the above. About two weeks after the start of stirring, various hydroxyapatite-suspended antitumor solutions were pulverized using Dynomill (Willy A. Baechofen AG Machinenfabrik Basel).

Pulverization was performed at a temperature of 5°C or less, using a circulation pump for cooling. In between pulverizations, particle size was measured (Microtrac 7340UPA particle size distribution analyzer, Nikkiso Co., Ltd.), and pulverization was completed when the maximum particle size became 100 nm or less; and various pulverized, hydroxyapatite-supplemented antitumor solutions were thus obtained. The maximum particle size varied slightly in each pulverization event, and was 0.03 to 0.1 µm (Example 1-1 to Example 1-11, and Example 1-20 to Example 1-33).

2% Hydroxyapatite-supplemented distilled water was pulverized using Dynomill (Willy A. Baechofen AG Machinenfabrik Basel). Pulverization was performed at a temperature of 5°C or less, using a circulation pump for cooling. In between pulverizations, particle size was measured (Microtrac 7340UPA particle size distribution analyzer, Nikkiso Co., Ltd.), and pulverization was completed when the maximum particle size became 0.1 µm or less, and particulate hydroxyapatite solutions were thus obtained. The maximum particle size was 0.07 µm.

Particulate hydroxyapatite solution was added to and mixed with fluorouracil, bleomycin hydrochloride, cisplatin, or mitomycin C, and each hydroxyapatite-supplemented antitumor agent was thus obtained. The amount of hydroxyapatite added was adjusted to be the same as for the hydroxyapatite-supplemented antitumor solutions produced above (Example 1-12 to Example 1-15).

Further, a given amount of fluorouracil, bleomycin hydrochloride, cisplatin, or mitomycin C was added to 5% hydroxyapatite-supplemented distilled water, followed by stirring, to produce various hydroxyapatite-suspended antitumor solutions. To accelerate the reaction, stirring was performed in darkness in a 50°C high temperature bath, and under reduced pressure to avoid any influence of air exposure.

About two weeks after the start of stirring, various hydroxyapatite-suspended antitumor solutions were pulverized using Dynomill (Willy A. Baechofen AG Machinenfabrik Basel).

Pulverization was performed at a temperature of 5°C or less, using a circulation pump for cooling. In between pulverizations, particle size was measured (Microtrac 7340UPA particle size distribution analyzer, Nikkiso Co., Ltd.), and pulverization was completed when the maximum particle size became 1 µm or less; and various pulverized, hydroxyapatite-supplemented antitumor solutions were thus obtained. The maximum particle size varied slightly in each pulverization event, and was 0.1 to 1 µm (Example 1-16 to Example 1-19).

Sobuzoxane was dissolved in chloroform and a given amount of the solution was added to 2% hydroxyapatite-supplemented distilled water, followed by stirring, to produce a hydroxyapatite-suspended antitumor solution.

To accelerate the reaction, stirring was performed in darkness in a 50°C high-temperature bath, and under reduced pressure to avoid any influence of air exposure.

About two weeks after the start of stirring, the hydroxyapatite-suspended antitumor solution was pulverized using Dynomill (Willy A. Baechofen AG Machinenfabrik Basel).

Pulverization was performed at a temperature of 5°C or less, using a circulation pump for cooling. In between pulverizations, particle size was measured (Microtrac 7340UPA particle size distribution analyzer, Nikkiso Co., Ltd.), and pulverization was completed when the maximum particle size became 0.1 µm or less; and a pulverized, hydroxyapatite-supplemented antitumor solution was thus obtained. The thus obtained antitumor solution was dried to obtain a solvent-free, pulverized, hydroxyapatite-supplemented sobuzoxane antitumor powder having a maximum particle size of 0.1 µm or less (Example 1-34).

Further, in between pulverizations, particle size was measured (Microtrac 7340UPA particle size distribution analyzer, Nikkiso Co., Ltd.), and the solution was removed when the maximum particle size became 10 µm or less, 5.0 µm or less, and 0.5 µm or less. Each antitumor solution was dried to remove the solvent, to obtain solvent-free, pulverized, hydroxyapatite-supplemented sobuzoxane antitumor powders having a maximum particle size of 10 µm or less (Example 1-37), 5.0 µm or less (Example 1-36), and 0.5 µm or less (Example 1-35).

Antitumor powders were produced according to methods similar to the above, except for the replacement of hydroxyapatite with calcium triphosphate, to obtain a solvent-free, pulverized, calcium triphosphate-supplemented sobuzoxane antitumor powder having a maximum particle size of 0.1 µm or less (Comparative Example 1-1).

Tretinoin was dissolved in dichloromethane, and a given amount of the solution was added to 2% hydroxyapatite-supplemented distilled water, followed by stirring, to produce a hydroxyapatite-suspended antitumor solution.

To accelerate the reaction, stirring was performed in darkness in a 50°C high temperature bath, and under reduced pressure to avoid any influence of air exposure.

About two weeks after the start of stirring, the hydroxyapatite-suspended antitumor solution was pulverized using Dynomill (Willy A. Baechofen AG Machinenfabrik Basel).

Pulverization was performed at a temperature of 5°C or less, using a circulation pump for cooling. In between pulverizations, particle size was measured (Microtrac 7340UPA particle size distribution analyzer, Nikkiso Co., Ltd.), and pulverization was completed when the maximum particle size became 0.1 µm or less, and a pulverized hydroxyapatite-supplemented tretinoin antitumor solution was obtained. The thus obtained antitumor solution was dried to obtain a solvent-free, pulverized, hydroxyapatite-supplemented tretinoin antitumor powder having a maximum particle size of 0.1 µm or less (Example 1-38).

A given amount of the tretinoin powder was added to 2% hydroxyapatite-supplemented distilled water, followed by stirring, to produce a hydroxyapatite-suspended tretinoin solution. To accelerate the reaction, stirring was performed in darkness in a 50°C high-temperature bath, and under reduced pressure to avoid any influence of air exposure.

About two weeks after the start of stirring, the hydroxyapatite-suspended antitumor solution was pulverized using Dynomill (Willy A. Baechofen AG Machinenfabrik Basel).

Pulverization was performed at a temperature of 5°C or less, using a circulation pump for cooling. In between pulverizations, particle size was measured (Microtrac 7340UPA particle size distribution analyzer, Nikkiso Co., Ltd.), and pulverization was completed when the maximum particle size became 0.1 µm or less, and a pulverized, hydroxyapatite-supplemented tretinoin antitumor solution having a maximum particle size of 0.1 µm or less was obtained (Example 1-39).

A given amount of water-soluble estramustine sodium phosphate, hydroxycarbamide, cytarabine ocfosfate, or doxifluridine, or water-insoluble toremifene citrate, mercaptopurine, or busulphan powder was added to 2% hydroxyapatite-supplemented distilled water followed by stirring, to produce various hydroxyapatite-suspended antitumor solutions. To accelerate the reaction, stirring was performed in darkness in a 50°C high-temperature bath, and under reduced pressure to avoid any influence of air exposure.

About two weeks after the start of stirring, various hydroxyapatite-suspended antitumor solutions were pulverized using Dynomill (Willy A. Baechofen AG Machinenfabrik Basel).

Pulverization was performed at a temperature of 5°C or less, using a circulation pump for cooling. In between pulverizations, particle size was measured (Microtrac 7340UPA particle size distribution analyzer, Nikkiso Co., Ltd.), and pulverization was completed when the maximum particle size became 5 µm or less for doxifluridine, busulphan, and nedaplatin. A pulverized, hydroxyapatite-supplemented doxifluridine antitumor solution having a maximum particle size of 5 µm or less (Example 1-47), a pulverized, hydroxyapatite-supplemented busulphan antitumor solution having a maximum particle size of 5 µm or less (Example 1-48), and a pulverized, hydroxyapatite-supplemented nedaplatin antitumor solution having a maximum particle size of 5 µm or less were obtained (Example 1-49). When the maximum particle size became 0.5 µm or less, pulverization was completed for hydroxycarbamide, cytarabine ocfosfate, and mercaptopurine. A pulverized, hydroxyapatite-supplemented hydroxycarbamide antitumor solution having a maximum particle size of 0.5 µm or less (Example 1-43), a pulverized, hydroxyapatite-supplemented cytarabine ocfosfate antitumor solution having a maximum particle size of 0.5 µm or less (Example 1-44), and a pulverized, hydroxyapatite-supplemented mercaptopurine antitumor solution having a maximum particle size of 0.5 µm or less (Example 1-45). Pulverization was completed for estramustine sodium phosphate and toremifene citrate when the maximum particle size became 0.1 µm or less. A pulverized, hydroxyapatite-supplemented estramustine sodium phosphate antitumor solution having a maximum particle size of 0.1 µm (Example 1-40) and a pulverized, hydroxyapatite-supplemented toremifene citrate antitumor solution having a maximum particle size of 0.1 µm (Example 1-41) were obtained.

Flutamide was dissolved in ethanol, tamoxifen citrate was dissolved in glacial acetic acid, and gefinitib was dissolved in dimethyl sulfoxide. A given amount of each solution was added to 2% hydroxyapatite-supplemented distilled water, followed by stirring, to produce hydroxyapatite-supplemented antitumor solutions of flutamide, tamoxifen citrate, and gefinitib.

To accelerate the reaction, stirring was performed in darkness in a 50°C high-temperature bath, and under reduced pressure to avoid any influence of air exposure.

About two weeks after the start of stirring, various hydroxyapatite-suspended antitumor solutions were pulverized using Dynomill (Willy A. Baechofen AG Machinenfabrik Basel).

Pulverization was performed at a temperature of 5°C or less, using a circulation pump for cooling. In between pulverizations, particle size was measured (Microtrac 7340UPA particle size distribution analyzer, Nikkiso Co., Ltd.), and pulverization was completed for tamoxifen citrate when the maximum particle size became 5 µm or less, and a pulverized, hydroxyapatite-supplemented antitumor tamoxifen citrate solution was thus obtained. For flutamide and gefinitib, pulverization was completed when the maximum particle size became 0.5 µm or less; and a pulverized, hydroxyapatite-supplemented, antitumor flutamide solution and a pulverized, hydroxyapatite-supplemented, antitumor gefinitib solution were obtained.

The thus obtained antitumor solutions were each dried to obtain a pulverized hydroxyapatite-supplemented tamoxifen citrate antitumor powder having a maximum particle size of 5 µm or less (Example 1-46), a solvent-free, hydroxyapatite-supplemented flutamide powder antitumor having a maximum particle size of 0.5 µm or less (Example 1-42), and a solvent-free, hydroxyapatite-supplemented gefinitib antitumor powder having a maximum particle size of 0.5 µm or less (Example 1-50).

2% Hydroxyapatite-supplemented distilled water was pulverized using Dynomill (Willy A. Baechofen AG Machinenfabrik Basel). Pulverization was performed at a temperature of 5°C or less, using a circulation pump for cooling. In between pulverizations, particle size was measured (Microtrac 7340UPA particle size distribution analyzer, Nikkiso Co., Ltd.), and pulverization was completed when the maximum particle size became 0.1 µm or less, and a particulate hydroxyapatite solution was thus obtained. The maximum particle size was 0.08 µm.

Particulate hydroxyapatite solution was added to and mixed with imatinib mesilate, oxaliplatin, UFT, carmofur, aceglatone, anastrozole, ubenimex, fadrozole hydrochloride hydrate, procarbazine hydrochloride, or bicalutamide to obtain hydroxyapatite-supplemented antitumor agents having a maximum particle size of 0.1 µm or less (Example 1-51 to Example 1-60).

The blending quantity of hydroxyapatite in each of the antitumor agents produced for toxicity tests is shown in Table 1 and Table 2.

**[Table 1]**

| Example | Antitumor agent | Hydroxyapatite load (%) | | | |
|---|---|---|---|---|---|
| | | -1 | -2 | -3 | -4 |
| Example 1-1 | Cyclophosphamide | 0.1 | 1.0 | 50 | 100 |
| Example 1-2 | Fluorouracil | 1.0 | 10 | 50 | 100 |
| Example 1-3 | Bleomycin hydrochloride | 1.0 | 10 | 50 | 200 |
| Example 1-4 | Etoposide | 1.0 | 50 | 100 | 200 |
| Example 1-5 | Vincristine sulfate | 0.1 | 1.0 | 50 | 200 |
| Example 1-6 | Interferon-β | 10 | 50 | 200 | 500 |
| Example 1-7 | Cisplatin | 1.0 | 10 | 50 | 200 |
| Example 1-8 | Carboplatin | 1.0 | 10 | 50 | 100 |
| Example 1-9 | Mitomycin C | 1.0 | 10 | 50 | 200 |
| Example 1-10 | Doxorubicin | 1.0 | 10 | 50 | 200 |
| Example 1-11 | Nimustine hydrochloride | 0.1 | 10 | 50 | 100 |
| Example 1-12 | Fluorouracil | 1.0 | 10 | 50 | 100 |
| Example 1-13 | Bleomycin hydrochloride | 1.0 | 10 | 50 | 200 |
| Example 1-14 | Cisplatin | 1.0 | 10 | 50 | 200 |
| Example 1-15 | Mitomycin C | 1.0 | 10 | 50 | 200 |
| Example 1-16 | Fluorouracil | 1.0 | 10 | 50 | 100 |
| Example 1-17 | Bleomycin hydrochloride | 1.0 | 10 | 50 | 200 |
| Example 1-18 | Cisplatin | 1.0 | 10 | 50 | 200 |
| Example 1-19 | Mitomycin C | 1.0 | 10 | 50 | 200 |
| Example 1-20 | Carboquone | 1.0 | 10 | 50 | 100 |
| Example 1-21 | Paclitaxel | 1.0 | 10 | 50 | 100 |
| Example 1-22 | Melphalan | 1.0 | 10 | 50 | 100 |
| Example 1-23 | Vinblastine sulfate | 1.0 | 10 | 50 | 100 |
| Example 1-24 | Dacarbazine | 10 | 50 | 200 | 500 |
| Example 1-25 | Ifosfamide | 10 | 50 | 200 | 500 |
| Example 1-26 | Thiotepa | 10 | 50 | 200 | 500 |
| Example 1-27 | Vinorelbine tartrate | 10 | 50 | 200 | 500 |
| Example 1-28 | Nedaplatin | 1.0 | 10 | 100 | 500 |
| Example 1-29 | Vinorelbine | 10 | 50 | 200 | 500 |
| Example 1-30 | Neocarzinostatin | 1.0 | 10 | 50 | 100 |
| Example 1-31 | Tegafur | 1.0 | 10 | 50 | 100 |
| Example 1-32 | Methotrexate | 1.0 | 10 | 50 | 200 |
| Example 1-33 | Goserelin acetate | 1.0 | 10 | 50 | 200 |
| Example 1-34 | Sobuzoxane | 10 | 100 | 500 | 1000 |
| Example 1-35 | Sobuzoxane | 10 | 100 | 500 | 1000 |
| Example 1-36 | Sobuzoxane | 10 | 100 | 500 | 1000 |
| Example 1-37 | Sobuzoxane | 10 | 100 | 500 | 1000 |

**[Table 2]**

| Example | Antitumor agent | Hydroxyapatite load (%) | | | |
|---|---|---|---|---|---|
| | | -1 | -2 | -3 | -4 |
| Example 1-38 | Tretinoin | 1.0 | 10 | 50 | 100 |
| Example 1-39 | Tretinoin | 1.0 | 10 | 50 | 100 |
| Example 1-40 | Estramustine sodium phosphate | 1.0 | 10 | 50 | 100 |
| Example 1-41 | Toremifene citrate | 1.0 | 10 | 50 | 100 |
| Example 1-42 | Flutamide | 1.0 | 10 | 50 | 100 |
| Example 1-43 | Hydroxycarbamide | 1.0 | 10 | 50 | 100 |
| Example 1-44 | Cytarabine ocfosfate | 1.0 | 10 | 100 | 500 |
| Example 1-45 | Mercaptopurine | 1.0 | 10 | 50 | 100 |
| Example 1-46 | Tamoxifen citrate | 1.0 | 10 | 50 | 100 |
| Example 1-47 | Doxifluridine | 1.0 | 10 | 50 | 100 |
| Example 1-48 | Busulphan | 1.0 | 10 | 50 | 100 |
| Example 1-49 | Nedaplatin | 1.0 | 10 | 100 | 500 |
| Example 1-50 | Gefinitib | 1.0 | 10 | 100 | 500 |
| Example 1-51 | Imatinib mesilate | 10 | 50 | 200 | 500 |
| Example 1-52 | Oxaliplatin | 0.1 | 1.0 | 50 | 100 |
| Example 1-53 | UFT | 0.1 | 1.0 | 50 | 100 |
| Example 1-54 | Carmofur | 0.1 | 1.0 | 50 | 200 |
| Example 1-55 | Aceglatone | 0.1 | 1.0 | 50 | 200 |
| Example 1-56 | Anastrozole | 1.0 | 10 | 50 | 100 |
| Example 1-57 | Ubenimex | 1.0 | 10 | 50 | 100 |
| Example 1-58 | Fadrozole hydrochloride hydrate | 1.0 | 10 | 50 | 200 |
| Example 1-59 | Procarbazine hydrochloride | 1.0 | 10 | 50 | 200 |
| Example 1-60 | Bicalutamide | 10 | 50 | 200 | 500 |
| Comparative Example 1-1 | Sobuzoxane | 10 | 100 | 500 | 1000 |

### [Test of toxicity-reducing effects by single administration of each antitumor agent]

Regarding samples in Example 1-1 to Example 1-9, Example 1-12 to Example 1-14, Example 1-16 to Example 1-18, and Example 1-20 to Example 1-28, each antitumor agent solution mixed with hydroxyapatite was intravascularly administered to a C57BL/6 male mouse (7 weeks old). After a one-time administration, observation was continued for 14 days, the number of deaths was confirmed, and LD50 was calculated from the dosage and number of deaths (Experimental Test 2-1 to Experimental Test 2-9, Experimental Test 2-12 to Experimental Test 2-14, Experimental Test 2-16 to Experimental Test 2-18, and Experimental Test 2-20 to Experimental Test 2-28). Example 1-29 to Example 1-32 were carried out by intraperitoneal administration, Example 1-33 by subcutaneous administration, and Example 1-34 to Example 1-49, Example 1-4, Example 1-7, Example 1-8, Example 1-19, Example 1-23, Example 1-25, Example 1-28, and Comparative Example 1-1 by oral administration. After a one-time administration, observation was continued for 14 days, the number of deaths was confirmed, and LD50 was calculated from the dosage and number of deaths (Experimental Test 2-29 to Experimental Test 2-56, and Comparative Example 2-1).

Oral administration of antitumor agents having a maximum particle size of 0.1 µm or less (Example 1-34, Example 1-38 to Example 1-41, Example 1-4, Example 1-7, Example 1-8, Example 1-23, Example 1-25, Example 1-28, and Comparative Example 1-1) was performed after emptying the mouse stomach, so that the antitumor agents pass through the mouse stomach before becoming dissolved by gastric acid. Antitumor agents having a maximum particle size of 0.5 µm or less (Example 1-35 and Example 1-42 to Example 1-45), 1 µm or less (Example 1-19), 5 µm or less (Example 1-36 and Example 1-46 to Example 1-49), and 10 µm or less (Example 1-37) were administered after sufficient feeding.

For comparison, similar tests, intravascular administration tests (Comparative Test 2-1 to Comparative Test 2-18), intraperitoneal administration tests (Comparative Test 2-19 to Comparative Test 2-22), subcutaneous administration tests (Comparative Test 2-23), and oral administration tests (Comparative Test 2-24 to Comparative Test 2-41) were carried out for the various antitumor agents.

The following test values indicate the applied dose as well as the number of animal deaths versus the number of administered animals (Experimental Tests 2-1-1 to 2-14-4, 2-34-1 to 2-34-4, 2-42-1 to 2-42-4, 2-44-1 to 2-44-4, Comparative Tests 2-1 to 2-8, 2-24, 2-28, and 2-30). Further, LD50 of the experimental tests and comparative tests for each antitumor agent is shown in Table 3 and Table 4.

### [Experimental Test 2-1-1] Pulverized 0.1% hydroxyapatite-supplemented cyclophosphamide solution

| | |
|---|---|
| 25 mg/kg | 0/5 |
| 50 | 1/6 |
| 125 | 2/6 |
| 150 | 3/5 |
| 225 | 5/6 |
| 250 | 5/8 |
| 275 | 6/6 |

### [Experimental Test 2-1-2] Pulverized 1.0% hydroxyapatite-supplemented cyclophosphamide solution

| | |
|---|---|
| 25 mg/kg | 0/8 |
| 50 | 1/6 |
| 125 | 2/6 |
| 150 | 3/5 |
| 225 | 4/6 |
| 250 | 5/8 |
| 300 | 6/6 |

### [Experimental Test 2-1-3] Pulverized 50% hydroxyapatite-supplemented cyclophosphamide solution

| | |
|---|---|
| 100 mg/kg | 0/6 |
| 150 | 1/6 |
| 200 | 2/6 |
| 250 | 2/5 |
| 300 | 3/5 |
| 400 | 4/6 |
| 500 | 4/6 |
| 600 | 5/5 |

### [Experimental Test 2-1-4] Pulverized 100% hydroxyapatite-supplemented cyclophosphamide solution

| | |
|---|---|
| 100 mg/kg | 0/6 |
| 150 | 1/6 |
| 200 | 2/6 |
| 250 | 2/5 |
| 300 | 3/5 |
| 400 | 3/5 |
| 500 | 4/6 |
| 600 | 5/5 |

### [Comparative Test 2-1] Cyclophosphamide

| | |
|---|---|
| 25 mg/kg | 0/6 |
| 50 | 1/5 |
| 125 | 2/6 |
| 150 | 3/6 |
| 200 | 4/6 |
| 250 | 4/5 |
| 300 | 6/6 |

### [Experimental Test 2-2-1] Pulverized 1.0% hydroxyapatite-supplemented fluorouracil solution

| | |
|---|---|
| 200 mg/kg | 0/6 |
| 300 | 1/5 |
| 400 | 3/6 |
| 500 | 4/6 |
| 600 | 5/6 |
| 700 | 6/6 |

### [Experimental Test 2-2-2] Pulverized 10% hydroxyapatite-supplemented fluorouracil solution

| | |
|---|---|
| 400 mg/kg | 0/5 |
| 500 | 1/6 |
| 600 | 1/5 |
| 700 | 2/6 |
| 800 | 3/5 |
| 900 | 5/8 |
| 1000 | 6/6 |

### [Experimental Test 2-2-3] Pulverized 50% hydroxyapatite-supplemented fluorouracil solution

| | |
|---|---|
| 500 mg/kg | 0/6 |
| 600 | 1/6 |
| 700 | 2/6 |
| 800 | 3/5 |
| 900 | 4/7 |
| 1000 | 4/5 |
| 1100 | 6/6 |

### [Experimental Test 2-2-4] Pulverized 100% hydroxyapatite-supplemented fluorouracil solution

| | |
|---|---|
| 400 mg/kg | 0/5 |
| 500 | 1/6 |
| 600 | 1/6 |
| 700 | 2/6 |
| 800 | 3/6 |
| 900 | 4/7 |
| 1000 | 4/5 |
| 1100 | 5/5 |

### [Comparative Test 2-2] Fluorouracil

| | |
|---|---|
| 50 mg/kg | 0/5 |
| 100 | 1/5 |
| 150 | 1/5 |
| 200 | 2/5 |
| 250 | 2/6 |
| 300 | 3/5 |
| 350 | 5/6 |
| 400 | 6/6 |

### [Experimental Test 2-3-1] Pulverized 1.0% hydroxyapatite-supplemented bleomycin hydrochloride solution

| | |
|---|---|
| 600 mg/kg | 0/6 |
| 700 | 1/6 |
| 800 | 2/6 |
| 900 | 4/5 |
| 1000 | 6/8 |
| 1200 | 6/7 |
| 1400 | 6/6 |

### [Experimental Test 2-3-2] Pulverized 10% hydroxyapatite-supplemented bleomycin hydrochloride solution

| | |
|---|---|
| 1000 mg/ kg | 0/6 |
| 1500 | 1/7 |
| 2000 | 2/7 |
| 2500 | 3/6 |
| 3000 | 3/5 |
| 3500 | 5/7 |
| 4000 | 4/5 |
| 4500 | 5/5 |

### [Experimental Test 2-3-3] Pulverized 50% hydroxyapatite-supplemented bleomycin hydrochloride solution

| | |
|---|---|
| 1000 mg/kg | 0/7 |
| 1500 | 1/7 |
| 2000 | 2/7 |
| 2500 | 2/6 |
| 3000 | 3/5 |
| 3500 | 4/6 |
| 4000 | 4/5 |
| 4500 | 6/6 |

### [Experimental Test 2-3-4] Pulverized 200% hydroxyapatite-supplemented bleomycin hydrochloride solution

| | |
|---|---|
| 1000 mg/kg | 0/6 |
| 1500 | 1/7 |
| 2000 | 1/6 |
| 2500 | 1/5 |
| 3000 | 3/6 |
| 3500 | 4/6 |
| 4000 | 6/7 |
| 4500 | 5/5 |

### [Comparative Test 2-3] Bleomycin hydrochloride

| | |
|---|---|
| 200 mg/ kg | 0/5 |
| 250 | 1/6 |
| 300 | 3/7 |
| 350 | 4/6 |
| 400 | 4/5 |
| 500 | 5/6 |
| 600 | 6/6 |

### [Experimental Test 2-4-1] Pulverized 1.0% hydroxyapatite-supplemented etoposide solution

| | |
|---|---|
| 30 mg/kg | 0/6 |
| 40 | 1/7 |
| 50 | 1/6 |
| 60 | 2/5 |
| 70 | 4/7 |
| 80 | 5/6 |
| 90 | 5/5 |

### [Experimental Test 2-4-2] Pulverized 50% hydroxyapatite-supplemented etoposide solution

| | |
|---|---|
| 150 mg/ kg | 0/6 |
| 200 | 1/8 |
| 250 | 1/6 |
| 300 | 2/5 |
| 400 | 5/7 |
| 450 | 5/6 |
| 500 | 6/6 |

### [Experimental Test 2-4-3] Pulverized 100% hydroxyapatite-supplemented etoposide solution

| | |
|---|---|
| 150 mg/ kg | 0/7 |
| 200 | 1/7 |
| 250 | 1/6 |
| 300 | 3/7 |
| 400 | 4/8 |
| 450 | 5/7 |
| 500 | 7/7 |

### [Experimental Test 2-4-4] Pulverized 200% hydroxyapatite-supplemented etoposide solution

| | |
|---|---|
| 150 mg/kg | 0/6 |
| 200 | 1/7 |
| 250 | 1/6 |
| 300 | 3/6 |
| 400 | 3/7 |
| 500 | 4/5 |
| 600 | 5/5 |

### [Comparative Test 2-4] Etoposide

| | |
|---|---|
| 10 mg/ kg | 0/6 |
| 20 | 1/6 |
| 30 | 2/6 |
| 40 | 5/7 |
| 50 | 5/6 |
| 60 | 6/6 |

### [Experimental Test 2-5-1] Pulverized 0.1% hydroxyapatite-supplemented vincristine sulfate solution

| | |
|---|---|
| 1.0 mg/kg | 0/5 |
| 1.5 | 2/5 |
| 2.0 | 3/8 |
| 2.5 | 3/5 |
| 3.0 | 6/7 |
| 3.5 | 4/5 |
| 4.0 | 6/6 |

### [Experimental Test 2-5-2] Pulverized 1.0% hydroxyapatite-supplemented vincristine sulfate solution

| | |
|---|---|
| 1.5 mg/kg | 0/5 |
| 2.0 | 1/6 |
| 2.5 | 3/5 |
| 3.0 | 6/8 |
| 3.5 | 6/8 |
| 4.0 | 7/8 |
| 4.5 | 6/6 |

### [Experimental Test 2-5-3] Pulverized 50% hydroxyapatite-supplemented vincristine sulfate solution

| | |
|---|---|
| 2.0 mg/kg | 0/6 |
| 3.0 | 1/6 |
| 4.0 | 1/5 |
| 5.0 | 2/5 |
| 6.0 | 3/6 |
| 7.0 | 5/6 |
| 8.0 | 5/5 |

### [Experimental Test 2-5-4] Pulverized 200% hydroxyapatite-supplemented vincristine sulfate solution

| | |
|---|---|
| 4.0 mg/kg | 0/7 |
| 5.0 | 1/8 |
| 6.0 | 1/6 |
| 7.0 | 1/6 |
| 8.0 | 2/6 |
| 9.0 | 4/6 |
| 10.0 | 4/5 |
| 11.0 | 5/5 |

### [Comparative Test 2-5] Vincristine sulfate

| | |
|---|---|
| 1.0 mg/kg | 0/5 |
| 1.5 | 2/5 |
| 2.0 | 3/8 |
| 2.5 | 4/6 |
| 3.0 | 7/8 |
| 3.5 | 8/8 |

### [Experimental Test 2-6-1] Pulverized 10% hydroxyapatite-supplemented interferon β solution

| | |
|---|---|
| 6000 mg/kg | 0/5 |
| 8000 | 1/6 |
| 10000 | 2/8 |
| 12000 | 3/7 |
| 14000 | 4/8 |
| 16000 | 4/5 |
| 18000 | 5/6 |
| 20000 | 6/6 |

### [Experimental Test 2-6-2] Pulverized 50% hydroxyapatite-supplemented interferon β solution

| | |
|---|---|
| 18000 mg/kg | 0/6 |
| 20000 | 1/6 |
| 22000 | 1/5 |
| 24000 | 3/6 |
| 26000 | 4/6 |
| 28000 | 4/5 |
| 30000 | 5/5 |

### [Experimental Test 2-6-3] Pulverized 200% hydroxyapatite-supplemented interferon β solution

| | |
|---|---|
| 15000 mg/kg | 0/5 |
| 20000 | 1/6 |
| 25000 | 2/6 |
| 30000 | 4/6 |
| 35000 | 5/6 |
| 40000 | 6/6 |

### [Experimental Test 2-6-4] Pulverized 500% hydroxyapatite-supplemented interferon β solution

| | |
|---|---|
| 20000 mg/kg | 0/6 |
| 22000 | 2/10 |
| 24000 | 2/8 |
| 26000 | 2/8 |
| 28000 | 3/8 |
| 30000 | 6/8 |
| 32000 | 8/9 |
| 33000 | 8/8 |

### [Comparative Test 2-6] Interferon β

| | |
|---|---|
| 600 mg/kg | 0/6 |
| 800 | 1/7 |
| 1000 | 2/6 |
| 1200 | 4/7 |
| 1500 | 5/8 |
| 1700 | 7/7 |

### [Experimental Test 2-7-1] Pulverized 1.0% hydroxyapatite-supplemented cisplatin solution

| | |
|---|---|
| 2.5 mg/kg | 0/5 |
| 5.0 | 1/6 |
| 10.0 | 3/6 |
| 15.0 | 6/7 |
| 20.0 | 5/6 |
| 25.0 | 5/5 |

### [Experimental Test 2-7-2] Pulverized 10% hydroxyapatite-supplemented cisplatin solution

| | |
|---|---|
| 2.5 mg/kg | 0/5 |
| 5.0 | 1/5 |
| 10.0 | 2/6 |
| 15.0 | 4/6 |
| 20.0 | 5/6 |
| 25.0 | 5/5 |

### [Experimental Test 2-7-3] Pulverized 50% hydroxyapatite-supplemented cisplatin solution

| | |
|---|---|
| 2.5 mg/kg | 0/6 |
| 5.0 | 1/6 |
| 10.0 | 2/6 |
| 15.0 | 3/6 |
| 20.0 | 5/6 |
| 25.0 | 6/6 |

### [Experimental Test 2-7-4] Pulverized 200% hydroxyapatite-supplemented cisplatin solution

| | |
|---|---|
| 2.5 mg/kg | 0/5 |
| 5.0 | 1/5 |
| 10.0 | 2/5 |
| 15.0 | 4/8 |
| 20.0 | 5/6 |
| 25.0 | 6/7 |
| 30.0 | 6/6 |

### [Comparative Test 2-7] Cisplatin

| | |
|---|---|
| 2.5 mg/kg | 0/5 |
| 5.0 | 1/8 |
| 7.5 | 2/7 |
| 10.0 | 5/8 |
| 12.5 | 6/8 |
| 15.0 | 5/6 |
| 17.5 | 6/6 |

### [Experimental Test 2-8-1] Pulverized 1.0% hydroxyapatite-supplemented carboplatin solution

| | |
|---|---|
| 70 mg/kg | 0/5 |

| | |
|---|---|
| 80 | 1/5 |
| 90 | 1/5 |
| 100 | 4/6 |
| 110 | 4/5 |
| 120 | 5/5 |

/

### [Experimental Test 2-8-2] Pulverized 10% hydroxyapatite-supplemented carboplatin solution

| | |
|---|---|
| 60 mg/kg | 0/6 |
| 80 | 1/6 |
| 100 | 3/6 |
| 120 | 5/6 |
| 140 | 6/7 |
| 160 | 6/6 |

### [Experimental Test 2-8-3] Pulverized 50% hydroxyapatite-supplemented carboplatin solution

| | |
|---|---|
| 60 mg/kg | 0/6 |
| 80 | 1/6 |
| 100 | 2/6 |
| 120 | 5/8 |
| 140 | 7/8 |
| 160 | 6/6 |

### [Experimental Test 2-8-4] Pulverized 100% hydroxyapatite-supplemented carboplatin solution

| | |
|---|---|
| 60 mg/kg | 0/6 |
| 80 | 1/8 |
| 100 | 2/6 |
| 120 | 5/7 |
| 140 | 7/8 |
| 160 | 8/8 |

### [Comparative Test 2-8] Carboplatin

| | |
|---|---|
| 70 mg/kg | 0/6 |
| 80 | 1/7 |
| 90 | 3/6 |
| 100 | 5/8 |
| 110 | 7/8 |
| 120 | 7/7 |

### [Experimental Test 2-12-1] 1.0% hydroxyapatite-supplemented fluorouracil solution

| | |
|---|---|
| 200 mg/kg | 1/6 |
| 300 | 1/5 |
| 400 | 2/5 |
| 500 | 4/6 |
| 600 | 5/6 |
| 700 | 6/6 |

### [Experimental Test 2-12-2] 10% hydroxyapatite-supplemented fluorouracil solution

| | |
|---|---|
| 100 mg/kg | 0/5 |
| 200 | 1/5 |
| 300 | 1/5 |
| 400 | 4/8 |
| 500 | 4/7 |
| 600 | 6/8 |
| 700 | 6/6 |

### [Experimental Test 2-12-3] 50% hydroxyapatite-supplemented fluorouracil solution

| | |
|---|---|
| 100 mg/kg | 0/7 |
| 200 | 1/6 |
| 300 | 1/5 |
| 400 | 3/8 |
| 500 | 3/6 |
| 600 | 5/7 |
| 700 | 5/5 |

### [Experimental Test 2-12-4] 100% hydroxyapatite-supplemented fluorouracil solution

| | |
|---|---|
| 200 mg/kg | 0/5 |
| 300 | 1/5 |
| 400 | 2/6 |
| 500 | 2/5 |
| 600 | 4/7 |
| 800 | 6/7 |
| 1000 | 6/6 |

### [Experimental Test 2-13-1] 1.0% hydroxyapatite-supplemented bleomycin sulfate solution

| | |
|---|---|
| 50 mg/kg | 0/5 |
| 100 | 1/8 |
| 200 | 2/8 |
| 400 | 3/8 |
| 600 | 4/7 |
| 800 | 6/8 |
| 1000 | 5/5 |

### [Experimental Test 2-13-2] 10% hydroxyapatite-supplemented bleomycin sulfate solution

| | |
|---|---|
| 600 mg/kg | 0/6 |
| 800 | 1/7 |
| 1000 | 1/5 |
| 1200 | 3/6 |
| 1400 | 3/5 |
| 1600 | 4/5 |
| 1800 | 6/6 |

### [Experimental Test 2-13-3] 50% hydroxyapatite-supplemented bleomycin sulfate solution

| | |
|---|---|
| 600 mg/kg | 0/5 |
| 800 | 1/7 |
| 1000 | 1/6 |
| 1200 | 2/6 |
| 1400 | 4/6 |
| 1600 | 4/5 |
| 1800 | 5/5 |

### [Experimental Test 2-13-4] 100% hydroxyapatite-supplemented bleomycin sulfate solution

| | |
|---|---|
| 600 mg/kg | 0/7 |
| 800 | 1/7 |
| 1000 | 1/6 |
| 1200 | 2/7 |
| 1400 | 4/7 |
| 1600 | 4/5 |
| 1800 | 7/7 |

### [Experimental Test 2-14-1] 1.0% hydroxyapatite-supplemented cisplatin solution

| | |
|---|---|
| 2.5 mg/kg | 0/6 |
| 5 | 1/6 |
| 10 | 4/7 |
| 15 | 6/8 |
| 20 | 7/8 |
| 25 | 6/6 |

### [Experimental Test 2-14-2] 10% hydroxyapatite-supplemented cisplatin solution

| | |
|---|---|
| 2.5 mg/kg | 0/6 |
| 5 | 1/6 |
| 10 | 2/6 |
| 15 | 5/7 |
| 20 | 7/8 |
| 25 | 6/6 |

### [Experimental Test 2-14-3] 50% hydroxyapatite-supplemented cisplatin solution

| | |
|---|---|
| 2.5 mg/kg | 0/5 |
| 5 | 1/6 |
| 10 | 2/6 |
| 15 | 4/6 |
| 20 | 5/6 |
| 25 | 6/7 |
| 30 | 5/5 |

### [Experimental Test 2-14-4] 100% hydroxyapatite-supplemented cisplatin solution

| | |
|---|---|
| 2.5 mg/kg | 0/5 |
| 5 | 1/5 |
| 10 | 2/6 |
| 15 | 4/6 |
| 20 | 6/7 |
| 25 | 7/8 |
| 30 | 7/7 |

### [Experimental Test 2-34-1] Pulverized 10% hydroxyapatite-supplemented sobuzoxane powder

| | |
|---|---|
| 2000 mg/kg | 0/5 |
| 2400 | 1/6 |
| 2800 | 1/6 |
| 3200 | 1/5 |
| 3400 | 2/5 |
| 3600 | 3/5 |
| 3800 | 4/5 |
| 4000 | 5/5 |

### [Experimental Test 2-34-2] Pulverized 100% hydroxyapatite-supplemented sobuzoxane powder

| | |
|---|---|
| 2400 mg/kg | 0/5 |
| 2800 | 1/6 |
| 3200 | 1/5 |
| 3600 | 2/6 |
| 4000 | 4/7 |
| 4400 | 4/5 |
| 4800 | 5/5 |

### [Experimental Test 2-34-3] Pulverized 500% hydroxyapatite-supplemented sobuzoxane powder

| | |
|---|---|
| 2000 mg/kg | 0/5 |
| 3000 | 1/7 |
| 4000 | 2/5 |
| 5000 | 3/5 |
| 6000 | 3/6 |
| 7000 | 4/5 |
| 8000 | 5/5 |

### [Experimental Test 2-34-4] Pulverized 1000% hydroxyapatite-supplemented sobuzoxane powder

| | |
|---|---|
| 3000 mg/kg | 0/5 |
| 4000 | 1/5 |
| 5000 | 1/5 |
| 6000 | 2/5 |
| 7000 | 3/5 |
| 8000 | 4/5 |
| 9000 | 5/5 |

### [Comparative Test 2-24] Sobuzoxane

| | |
|---|---|
| 200 mg/kg | 0/5 |
| 400 | 1/5 |
| 600 | 1/6 |
| 800 | 2/5 |
| 1000 | 3/5 |
| 1200 | 4/5 |
| 1400 | 6/6 |

### [Experimental Test 2-42-1] Pulverized 1.0% hydroxyapatite-supplemented flutamide powder

| | |
|---|---|
| 2000 mg/kg | 0/5 |
| 3000 | 1/5 |
| 3200 | 2/5 |
| 3400 | 3/5 |
| 3600 | 3/5 |
| 3800 | 4/5 |
| 4000 | 4/5 |
| 4200 | 5/5 |

### [Experimental Test 2-42-2] Pulverized 10% hydroxyapatite-supplemented flutamide powder

| | |
|---|---|
| 3000 mg/kg | 0/5 |
| 3200 | 1/5 |
| 3400 | 1/5 |
| 3600 | 2/5 |
| 3800 | 3/5 |
| 4000 | 4/5 |
| 4200 | 5/5 |

### [Experimental Test 2-42-3] Pulverized 50% hydroxyapatite-supplemented flutamide powder

| | |
|---|---|
| 4200 mg/kg | 0/5 |
| 4400 | 1/5 |
| 4600 | 1/5 |
| 4800 | 2/5 |
| 5000 | 3/5 |
| 5200 | 3/5 |
| 5400 | 4/5 |
| 5600 | 5/5 |

### [Experimental Test 2-42-4] Pulverized 100% hydroxyapatite-supplemented flutamide powder

| | |
|---|---|
| 4600 | 0/5 |
| 4800 | 1/5 |
| 5000 | 1/5 |
| 5200 | 2/5 |
| 5400 | 2/5 |
| 5600 | 4/5 |
| 5800 | 5/5 |

### [Comparative Test 2-28] Flutamide

| | |
|---|---|
| 250 mg/kg | 0/5 |
| 500 | 1/5 |
| 750 | 3/5 |
| 1000 | 3/5 |
| 1250 | 4/5 |
| 1500 | 4/5 |
| 2000 | 5/5 |

### [Experimental Test 2-44-1] Pulverized 1.0% hydroxyapatite-supplemented cytarabine ocfosfate powder

| | |
|---|---|
| 500 mg/kg | 0/5 |
| 750 | 1/5 |
| 1000 | 1/5 |
| 1250 | 2/5 |
| 1500 | 3/5 |
| 1750 | 3/5 |
| 2000 | 4/5 |
| 2250 | 5/5 |

### [Experimental Test 2-44-2] Pulverized 10% hydroxyapatite-supplemented cytarabine ocfosfate powder

| | |
|---|---|
| 1250 mg/kg | 0/5 |
| 1500 | 1/5 |
| 1750 | 2/5 |
| 2000 | 3/5 |
| 2250 | 3/5 |
| 2500 | 4/5 |
| 2750 | 4/5 |
| 3000 | 5/5 |

### [Experimental Test 2-44-3] Pulverized 50% hydroxyapatite-supplemented cytarabine ocfosfate powder

| | |
|---|---|
| 1750 mg/kg | 0/5 |
| 2000 | 1/5 |
| 2250 | 2/5 |
| 2500 | 2/5 |
| 2750 | 2/5 |
| 3000 | 4/5 |
| 3250 | 4/5 |
| 3500 | 5/5 |

### [Experimental Test 2-44-4] Pulverized 100% hydroxyapatite-supplemented cytarabine ocfosfate powder

| | |
|---|---|
| 2000 mg/kg | 0/5 |
| 2250 | 1/5 |
| 2500 | 2/5 |
| 2750 | 2/5 |
| 3000 | 3/5 |
| 3250 | 3/5 |
| 3500 | 4/5 |
| 3750 | 5/5 |

### [Comparative Test 2-30] Cytarabine ocfosfate

| | |
|---|---|
| 250 mg/kg | 0/5 |
| 300 | 1/5 |
| 400 | 2/5 |
| 500 | 2/5 |
| 600 | 3/5 |
| 700 | 3/5 |
| 800 | 4/5 |
| 1000 | 5/5 |

**[Table 3]**

| [LD50 of single-administration tests] 1 | | | | | |
|---|---|---|---|---|---|
| Test | Antitumor agent | LD50 (mg/kg) | | | |
| | | -1 | -2 | -3 | -4 |
| Experimental Test 2-1 | Cyclophosphamide | 141.5 | 155.5 | 298.2 | 307.4 |
| Experimental Test 2-2 | Fluorouracil | 411.5 | 785.5 | 798.3 | 802.7 |
| Experimental Test 2-3 | Bleomycin hydrochloride | 853.2 | 2603.8 | 2749.5 | 2888.9 |
| Experimental Test 2-4 | Etoposide | 63.4 | 325.7 | 362.4 | 366.7 |
| Experimental Test 2-5 | Vincristine sulfate | 2.0 | 2.5 | 5.3 | 8.3 |
| Experimental Test 2-6 | Interferon-β | 12771 | 24241 | 26985 | 27401 |
| Experimental Test 2-7 | Cisplatin | 9.3 | 11.1 | 12.4 | 11.7 |
| Experimental Test 2-8 | Carboplatin | 95.9 | 100.4 | 107.8 | 106.8 |
| Experimental Test 2-9 | Mitomycin C | 11.3 | 28.5 | 49.1 | 72.6 |
| Experimental Test 2-12 | Fluorouracil | 402.1 | 414.7 | 465.9 | 511.8 |
| Experimental Test 2-13 | Bleomycin hydrochloride | 452.9 | 1233.5 | 1270.0 | 1321.5 |
| Experimental Test 2-14 | Cisplatin | 9.3 | 10.7 | 11.3 | 10.9 |
| Experimental Test 2-16 | Fluorouracil | 278.1 | 289.1 | 325.8 | 353.0 |
| Experimental Test 2-17 | Bleomycin hydrochloride | 365.3 | 406.7 | 452.6 | 487.4 |
| Experimental Test 2-18 | Cisplatin | 9.2 | 9.9 | 10.8 | 10.5 |
| Experimental Test 2-20 | Carboquone | 61.8 | 103 | 132 | 146 |
| Experimental Test 2-21 | Paclitaxel | 94 | 157 | 229 | 241 |
| Experimental Test 2-22 | Melphalan | 258 | 415 | 576 | 592 |
| Experimental Test 2-23 | Vinblastine sulfate | 547 | 662 | 871 | 919 |
| Experimental Test 2-24 | Dacarbazine | 2011 | 2577 | 3869 | 4054 |
| Experimental Test 2-25 | Ifosfamide | 1546 | 2280 | 3415 | 3473 |
| Experimental Test 2-26 | Thiotepa | 92 | 216 | 387 | 417 |
| Experimental Test 2-27 | Vinorelbine tartrate | 58 | 201 | 425 | 431 |
| Experimental Test 2-28 | Nedaplatin | 42 | 44 | 47 | 49 |
| Experimental Test 2-29 | Vinorelbine | 295 | 573 | 892 | 1257 |
| Experimental Test 2-30 | Neocarzinostatin | 3627 | 5864 | 6953 | 7524 |
| Experimental Test 2-31 | Tegafur | 6249 | 8976 | >10000 | >10000 |
| Experimental Test 2-32 | Methotrexate | 382 | 459 | 688 | 725 |
| Experimental Test 2-33 | Goserelin acetate | 304 | 417 | 492 | 526 |
| Experimental Test 2-34 | Sobuzoxane | 3453 | 3797 | 5166 | 4963 |
| Experimental Test 2-35 | Sobuzoxane | 3512 | 4124 | 5951 | 5907 |
| Experimental Test 2-36 | Sobuzoxane | 3046 | 3280 | 4179 | 4006 |
| Experimental Test 2-37 | Sobuzoxane | 2100 | 2251 | 2197 | 2015 |
| Experimental Test 2-38 | Tretinoin | 2385 | 4530 | 6718 | 7215 |
| Experimental Test 2-39 | Tretinoin | 2253 | 4381 | 6472 | 6859 |
| Experimental Test 2-40 | Estramustine sodium phosphate | 1157 | 2251 | 2640 | 2735 |
| | | | | | |

| [LD50 of single-administration tests] 2 | | | | | |
|---|---|---|---|---|---|
| Test | Antitumor agent | LD50 (mg/kg) | | | |
| | | -1 | -2 | -3 | -4 |
| Experimental Test 2-41 | Toremifene citrate | 710 | 1285 | 1561 | 1587 |
| Experimental Test 2-42 | Flutamide | 3429 | 3670 | 4946 | 5341 |
| Experimental Test 2-43 | Hydroxycarbamide | 8916 | >10000 | >10000 | >10000 |
| Experimental Test 2-44 | Cytarabine ocfosfate | 1385 | 1945 | 2590 | 2845 |
| Experimental Test 2-45 | Mercaptopurine | 3559 | 6097 | 7256 | 8301 |
| Experimental Test 2-46 | Tamoxifen citrate | 5310 | 6823 | 7418 | 7609 |
| Experimental Test 2-47 | Doxifluridine | 7510 | 8827 | >10000 | >10000 |
| Experimental Test 2-48 | Busulphan | 75 | 141 | 175 | 184 |
| Experimental Test 2-49 | Nedaplatin | 516 | 1231 | 1772 | 2187 |
| Experimental Test 2-50 | Etoposide | 515 | 1233 | 1498 | 2071 |
| Experimental Test 2-51 | Cisplatin | 102 | 449 | 735 | 867 |
| Experimental Test 2-52 | Carboplatin | 1181 | 2130 | 3092 | 3684 |
| Experimental Test 2-53 | Mitomycin C | 87 | 245 | 356 | 421 |
| Experimental Test 2-54 | Vinblastine sulfate | 1390 | 3841 | 4538 | 4704 |
| Experimental Test 2-55 | Ifosfamide | 3626 | 4359 | 4725 | 5287 |
| Experimental Test 2-56 | Nedaplatin | 592 | 1397 | 1919 | 2347 |
| Comparative Example 2-1 | Sobuzoxane | 959 | 1087 | 1110 | 1076 |

**[Table 4]**

| Test | Antitumor agent | LD50 (mg/kg) |
|---|---|---|
| Comparative Test 2-1 | Cyclophosphamide | 140.5 |
| Comparative Test 2-2 | Fluorouracil | 243.6 |
| Comparative Test 2-3 | Bleomycin hydrochloride | 322.7 |
| Comparative Test 2-4 | Etoposide | 32.7 |
| Comparative Test 2-5 | Vincristine sulfate | 1.98 |
| Comparative Test 2-6 | Interferon-β | 1222 |
| Comparative Test 2-7 | Cisplatin | 9.1 |
| Comparative Test 2-8 | Carboplatin | 93.0 |
| Comparative Test 2-9 | Mitomycin C | 4.7 |
| Comparative Test 2-10 | Carboquone | 5.8 |
| Comparative Test 2-11 | Paclitaxel | 12 |
| Comparative Test 2-12 | Melphalan | 21 |
| Comparative Test 2-13 | Vinblastine sulfate | 5.6 |
| Comparative Test 2-14 | Dacarbazine | 452 |
| Comparative Test 2-15 | Ifosfamide | 325 |
| Comparative Test 2-16 | Thiotepa | 16.1 |
| Comparative Test 2-17 | Vinorelbine tartrate | 2.3 |
| Comparative Test 2-18 | Nedaplatin | 41 |
| Comparative Test 2-19 | Vinorelbine | 29 |
| Comparative Test 2-20 | Neocarzinostatin | 1050 |
| Comparative Test 2-21 | Tegafur | 750 |
| Comparative Test 2-22 | Methotrexate | 74 |
| Comparative Test 2-23 | Goserelin acetate | 36 |
| Comparative Test 2-24 | Sobuzoxane | 853.2 |
| Comparative Test 2-25 | Tretinoin | 549 |
| Comparative Test 2-26 | Estramustine sodium phosphate | 380 |
| Comparative Test 2-27 | Toremifene citrate | 360 |
| Comparative Test 2-28 | Flutamide | 772 |
| Comparative Test 2-29 | Hydroxycarbamide | 2350 |
| Comparative Test 2-30 | Cytarabine ocfosfate | 524 |
| Comparative Test 2-31 | Mercaptopurine | 1094 |
| Comparative Test 2-32 | Tamoxifen citrate | 1240 |
| Comparative Test 2-33 | Doxifluridine | 1200 |
| Comparative Test 2-34 | Busulphan | 40 |
| Comparative Test 2-35 | Nedaplatin | 140 |
| Comparative Test 2-36 | Etoposide | 220 |
| Comparative Test 2-37 | Cisplatin | 30 |
| Comparative Test 2-38 | Carboplatin | 350 |
| Comparative Test 2-39 | Mitomycin C | 25 |
| Comparative Test 2-40 | Vinblastine sulfate | 427 |
| Comparative Test 2-41 | Ifosfamide | 996 |

### [Test of toxicity-reducing effects by repetitive administration of each antitumor agent]

For samples of Example 1-9 to 1-11, 1-15, and 1-19, each hydroxyapatite-blended antitumor agent solution was subcutaneously administered repetitively to the hind leg of a BDF₁ mouse transplanted with 10⁶ Lewis lung cancer cells, from Day 6 of the tumor transplantation. The dosage which killed 50% or more by Day 9 of the administration was regarded as LD50 (Experimental Test 3-9 to 3-11, Experimental Test 3-15, and Experimental Test 3-19). For comparison, similar tests were performed with each of the antitumor agents (Comparative Test 3-1 to 3-3). LD50 of the repetitive-administration tests is shown in Table 5.

**[Table 5]**

| [LD50 of repetitive-administration tests] | | | | | |
|---|---|---|---|---|---|
| Test | Antitumor agent | LD50 (mg/kg) | | | |
| | | -1 | -2 | -3 | -4 |
| Experimental Test 3-9 | Mitomycin C | 9 | 20 | 38 | 54 |
| Experimental Test 3-10 | Doxorubicin | 17 | 25 | 42 | 49 |
| Experimental Test 3-11 | Nimustine hydrochloride | 57 | 87 | 106 | 110 |
| Experimental Test 3-15 | Mitomycin C | 7 | 14 | 21 | 25 |
| Experimental Test 3-19 | Mitomycin C | 7 | 17 | 29 | 41 |
| Comparative Test 3-1 | Mitomycin C | 3 | | | |
| Comparative Test 3-2 | Doxorubicin | 10 | | | |
| Comparative Test 3-3 | Nimustine hydrochloride | 50 | | | |

The above results show that the toxicity of all the hydroxyapatite-supplemented antitumor agents was reduced, as compared with the toxicity of the antitumor agents. In particular, toxicity was dramatically reduced in antitumor agents produced by mixing antitumor agents with hydroxyapatite followed by pulverization.

For comparison, tests using other calcium phosphates were performed, and a slight reduction of toxicity in pulverized, tricalcium phosphate-supplemented sobuzoxane antitumor agents was observed. However, the reduction was not as great as that observed with pulverized hydroxyapatite-supplemented sobuzoxane antitumor agents.

### [Cell growth inhibition tests]

Cell growth inhibition tests were performed *in vitro,* for samples of Examples 1-4, 1-5, 1-7, 1-8, 1-9, 1-10, 1-12, and 1-16 (Experimental Tests 4-1 to 4-8).

The tests were performed using 15 types of human cancer cells: HeLa and SiHa for cervical cancer; HT1080 for fibrosarcoma; A431 for squamous cell carcinoma; HSC3, HSC4, and KB for oral cancer; AZ521, Kato-III, and NUGC-4 for stomach cancer; SW837 and LoVo for colon cancer; HepG2 for liver cancer; Lu65 and Lu99 for lung cancer, and the average 50% growth inhibition parameter (GI 50) was calculated by the following procedures. Appropriate amounts of each of the human cancer cells suspended in 5% fetal bovine serum-supplemented medium were plated into a 96-well plate, and allowed to adhere to the wells in a 37°C CO₂ incubator overnight.

These cancer cells were exposed to each anticancer agent for 48 hours. Cell growth was assayed by MTT. Absorbance of the control well (C) and the test well (T) was measured at 550 nm.

The absorbance of the test well immediately after adding the drug (TO) was also measured. Using these measured values, GI 50 of each drug was calculated, and the drug concentration at that time was obtained. For comparison, a similar test was performed for each of the anticancer agents (Comparative Tests 4-1 to 4-7).

GI 50 determined by the cell growth inhibition tests is shown in Table 6.

**[Table 6]**

| [Cell growth inhibition test results] Average GI 50 determined by the cell growth inhibition tests | | | | | |
|---|---|---|---|---|---|
| Test | Antitumor agent | GI 50 (µm) | | | |
| | | -1 | -2 | -3 | -4 |
| Experimental Test 4-1 | Etoposide | 5.03 | 4.82 | 4.67 | 4.49 |
| Experimental Test 4-2 | Vincristine sulfate | 0.0033 | 0.0031 | 0.0026 | 0.0021 |
| Experimental Test 4-3 | Cisplatin | 7.06 | 6.94 | 6.73 | 6.29 |
| Experimental Test 4-4 | Carboplatin | 75.25 | 70.26 | 67.99 | 65.04 |
| Experimental Test 4-5 | Mitomycin C | 1.30 | 1.19 | 1.14 | 1.02 |
| Experimental Test 4-6 | Doxorubicin | 0.060 | 0.049 | 0.043 | 0.034 |
| Experimental Test 4-7 | Fluorouracil | 34.56 | 32.06 | 30.25 | 29.04 |
| Experimental Test 4-8 | Fluorouracil | 35.04 | 33.10 | 32.57 | 30.86 |
| Comparative Test 4-1 | Etoposide | 5.11 | | | |
| Comparative Test 4-2 | Vincristine sulfate | 0.0036 | | | |
| Comparative Test 4-3 | Cisplatin | 7.21 | | | |
| Comparative Test 4-4 | Carboplatin | 75.68 | | | |
| Comparative Test 4-5 | Mitomycin C | 1.43 | | | |
| Comparative Test 4-6 | Doxorubicin | 0.072 | | | |
| Comparative Test 4-7 | Fluorouracil | 36.82 | | | |

Cell growth inhibition tests similar to the above were performed using samples from Examples 1-20 to 1-32 which test the toxicity-reducing effect by single administration of various antitumor agents. For comparison, a similar test was performed for each anticancer agent. The results showed that, similar to the results shown in Table 6, GI 50 of hydroxyapatite-supplemented antitumor agents was slightly smaller than the GI 50 of the comparative antitumor agents.

### [Antitumor tests]

Antitumor tests were performed by the methods described below, using cyclophosphamide, fluorouracil, etoposide, vincristine sulfate, and cisplatin, each of which was prepared by adsorption with 10% hydroxyapatite particles with a maximum particle size of 50 nm, using the method described above [Preparation of various hydroxyapatite-supplemented antitumor agent solutions] (Experimental Test 5-1 to 5-5). Fluorouracil and cisplatin were each adsorbed with 10% hydroxyapatite with a maximum particle size of 0.8 µm, and the antitumor tests were performed by similar methods (Experimental Test 5-6 and 5-7).

Female nude mice (7 weeks old, 16 to 22g) with a BALB/c genetic background were used for the tests. Human cancer cells used for the tests were GCIY stomach cancer cells, HCT-15 colon cancer cells, A549 lung cancer cells, and OVCAR-3 ovarian cancer cells. Human cancer cells were subcutaneously transplanted into nude mice, tumor lumps were formed, and these carcinomas were used for the tests.

Aseptically collected tumors were sliced into tumors of 3 x 3 x 3 mm, and then subcutaneously transplanted into a new nude mouse. When the tumors reached a volume of 100 to 300 mm³, the animals were arbitrarily divided into groups of six (Day 0). Administration was started from Day 0 according to the following administration schedule: single administration of cyclophosphamide at a dosage of 260 mg/kg; triple administration of fluorouracil at a dosage of 50 mg/kg at four-day intervals; consecutive administration of etoposide at 12 mg/kg for 5 days; single administration of vincristine sulfide at 1.6 mg/kg; and single administration of cisplatin at 10 mg/kg. Intravascular administration was the administration route for all.

After administration, between Days 24 to 31, the length (L) and width (W) of the tumor lump of the animal were measured twice a week, and the tumor volume (TV) was calculated according to the following formula: TV = (L x W²)/2. Antitumor effect was determined at Day 14. The relative tumor volume (RTV) was obtained by the following formula: RTV = TV14/TV0, using values of the tumor volume at Day 0 and Day 14 (referred to as TV0 and TV 14). Antitumor effect was evaluated as the tumor regression rate at Day 14 (T/C%). Herein, T/C% = 100 x (average RTV of the treated group)/(RTV of the control group).

For comparison, cyclophosphamide, fluorouracil, etoposide, vincristine sulfate, or cisplatin was adsorbed with 10% hydroxyapatite particles with a particle size of 30 to 50 µm and antitumor effects were tested with an administration schedule and administration dosage identical to the above, by intraperitoneal administration (Comparative Tests 5-1 to 5-5).

Further as a comparison, cyclophosphamide, fluorouracil, vincristine sulfate or cisplatin was adsorbed with 10% hydroxyapatite particles with a particle size of about 150 to 250 µm, and then implanted into a tumor site to test antitumor effects (Comparative Tests 5-6 to 5-9). As a control, each drug was tested without hydroxyapatite adsorption (Comparative Tests 5-10 to 5-14). The regression rate (%) of each transplanted cancer in the antitumor tests is shown in Table 7.

**[Table 7]**

| [Antitumor test results] Regression rate of each transplanted cancer. | | | | | |
|---|---|---|---|---|---|
| Test | Antitumor agent | GCIY | HCT-15 | A549 | OVCAR-3 |
| Experimental Test 5-1 | Cyclophosphamide | 64 | 37 | 70 | 56 |
| Experimental Test 5-2 | Fluorouracil | 54 | 42 | 87 | 54 |
| Experimental Test 5-3 | Etoposide | 79 | 54 | 58 | 62 |
| Experimental Test 5-4 | Vincristine sulfate | 68 | 79 | 57 | 61 |
| Experimental Test 5-5 | Cisplatin | 60 | 46 | 71 | 62 |
| Experimental Test 5-6 | Fluorouracil | 54 | 42 | 87 | 54 |
| Experimental Test 5-7 | Cisplatin | 62 | 46 | 74 | 61 |
| Comparative Test 5-1 | Cyclophosphamide | 79 | 55 | 85 | 73 |
| Comparative Test 5-2 | Fluorouracil | 71 | 61 | 118 | 77 |
| Comparative Test 5-3 | Etoposide | 98 | 70 | 76 | 79 |
| Comparative Test 5-4 | Vincristine sulfate | 83 | 105 | 79 | 84 |
| Comparative Test 5-5 | Cisplatin | 66 | 59 | 84 | 69 |
| Comparative Test 5-6 | Cyclophosphamide | 83 | 61 | 91 | 79 |
| Comparative Test 5-7 | Fluorouracil | 73 | 67 | 123 | 80 |
| Comparative Test 5-8 | Vincristine sulfate | 92 | 117 | 86 | 91 |
| Comparative Test 5-9 | Cisplatin | 73 | 62 | 90 | 79 |
| Comparative Test 5-10 | Cyclophosphamide | 78 | 52 | 86 | 71 |
| Comparative Test 5-11 | Fluorouracil | 69 | 59 | 117 | 74 |
| Comparative Test 5-12 | Etoposide | 93 | 67 | 72 | 76 |
| Comparative Test 5-13 | Vincristine sulfate | 81 | 101 | 76 | 79 |
| Comparative Test 5-14 | Cisplatin | 67 | 53 | 79 | 67 |

The above results show that intravascular administration of antitumor solutions of various antitumor agents added with hydroxyapatite microparticles can reduce toxicity without reducing the effects of the various antitumor agents.

### [Cell growth inhibition tests]

Cell growth inhibition tests were performed *in vitro,* using samples of Example 1-34 (sobuzoxane), Example 1-42 (flutamide), Example 1-2 (fluorouracil), Example 1-1 (cyclophosphamide), Example 1-44 (cytarabine ocfosfate), Example 1-50 (gefinitib), and Example 1-4 (etoposide).

The average 50% growth inhibition parameter (IC50) was calculated for the sample of Example 1-34 by the procedure shown below, using Colon26 colon cancer cells and Lewis lung cancer cells. Colon26 colon cancer cells or Lewis lung cancer cells were suspended in a medium containing 5% fetal bovine serum and adjusted to 10⁴ cells/ml. 100 µl of the cells was plated into each well of a 96-well plate, and then allowed to adhere to the wells in a 37°C CO₂ incubator overnight.

These cancer cells were exposed to each anticancer agent for 48 hours. Cell growth was assayed by MTT. Absorbance of the control well (C) and the test well (T) was measured at 550 nm.

IC50 of each drug was calculated using these measured values, and the drug concentration was obtained (Experimental Test 6-1 and Experimental Test 6-2). For comparison, a similar test was performed for sobuzoxane (Comparative Test 6-1 and Comparative Test 6-2). IC50 determined by the cell growth inhibition test is shown in Table 8.

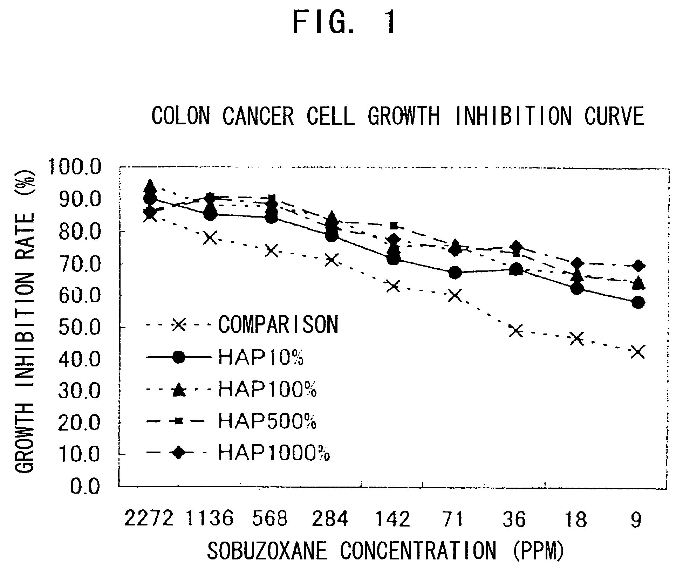

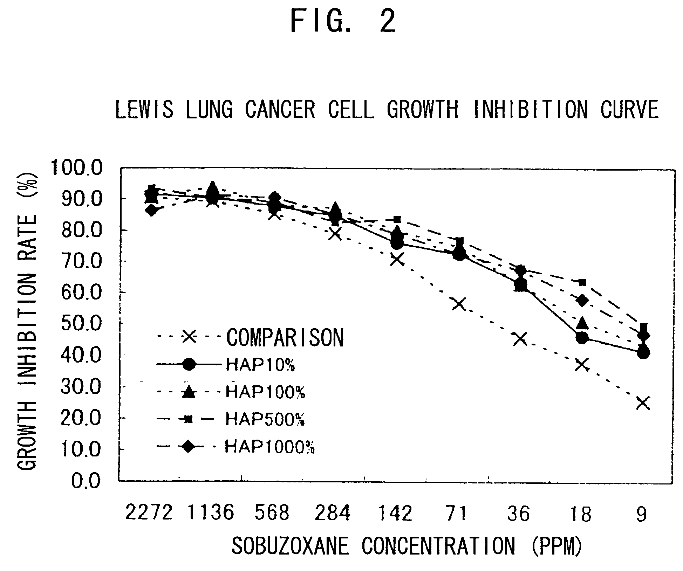

The cell growth inhibition curves determined by the cell growth inhibition test using Colon26 colon cancer cells are shown in Fig. 1, and the cell growth inhibition curves determined by the cell growth inhibition test using Lewis lung cancer cells are shown in Fig. 2.

### [Test samples]

Comparison: sobuzoxane, HAP 10%: Example 1-34-1, HAP 100%: Example 1-34-2, HAP 500%: Example 1-34-3, HAP 1000%: Example 1-34-4

**[Table 8]**

| [Cell growth inhibition test result 1] Average IC50 of the hydroxyapatite-supplemented sobuzoxane antitumor agent determined by the cell growth inhibition test | | | | | |
|---|---|---|---|---|---|
| Cancer cell | Test | IC50 (ppm) | | | |
| | | -1 | -2 | -3 | -4 |
| Colon26 colonic cancer cell | Experimental Test 6-1 | 5.03 | 2.35 | 0.96 | 0.28 |
| | Comparative Test 6-1 | 25.45 | | | |
| Lewis lung cancer cell | Experimental Test 6-2 | 14.83 | 11.86 | 5.52 | 6.86 |
| | Comparative Test 6-2 | 42.42 | | | |

*In vitro* cell growth inhibition test was performed with the sample of Example 1-42 by methods similar to the above, using Lewis lung cancer cells, P388 leukemia cells, and DU 145 prostate cancer cells. Similar to the above, IC50 of the drug was calculated using these measured values, and the drug concentration was obtained (Experimental Test 6-3 to Experimental Test 6-5). As a comparison, a similar test was performed for flutamide (Comparative Test 6-3 to Comparative Test 6-5). IC50 determined by the cell growth inhibition test is shown in Table 9.

**[Table 9]**

| [Cell growth inhibition test result 2] Average IC50 of the hydroxyapatite-supplemented flutamide antitumor agent determined by the cell growth inhibition test | | | | | |
|---|---|---|---|---|---|
| Cancer cell | Test | IC50 (ppm) | | | |
| | | -1 | -2 | -3 | -4 |
| Lewis lung cancer cell | Experimental Test 6-3 | 51.0 | 32.7 | 20.1 | 15.6 |
| | Comparative Test 6-3 | 75.3 | | | |
| P388 leukemia cell | Experimental Test 6-4 | 44.9 | 25.5 | 14.0 | 10.2 |
| | Comparative Test 6-4 | 107.8 | | | |
| DU145 prostate cancer cell | Experimental Test 6-5 | 32.6 | 17.3 | 9.7 | 4.1 |
| | Comparative Test 6-5 | 126.8 | | | |

*In vitro* cell growth inhibition test was performed with the sample of Example 1-2 by methods similar to the above using Colon26 colon cancer cells, Lewis lung cancer cells, and P388 leukemia cells. Similar to the above, IC50 of the drug was calculated using these measured values, and the drug concentration was obtained (Experimental Test 6-6 to Experimental Test 6-8). As a comparison, similar tests were performed for fluorouracil (Comparative Test 6-6 to Comparative Test 6-8). IC50 determined by the cell growth inhibition tests is shown in Table 10.

**[Table 10]**

| [Cell growth inhibition test result 3] Average IC50 of the hydroxyapatite-supplemented fluorouracil antitumor agent determined by the cell growth inhibition test | | | | | |
|---|---|---|---|---|---|
| Cancer cell | Test | IC50 (ppm) | | | |
| | | -1 | -2 | -3 | -4 |
| Colon26 colonic cancer cell | Experimental Test 6-6 | 0.11 | 0.08 | 0.05 | 0.02 |
| | Comparative Test 6-6 | 0.16 | | | |
| Lewis lung cancer cell | Experimental Test 6-7 | 0.22 | 0.14 | 0.06 | 0.03 |
| | Comparative Test 6-7 | 0.3 8 | | | |
| P388 leukemia cell | Experimental Test 6-8 | 2.76 | 0.90 | 0.12 | 0.06 |
| | Comparative Test 6-8 | 6.07 | | | |

Cell growth inhibition test was performed *in vitro* with the samples of Example 1-1, Example 1-44, and Example 1-50 by methods similar to the above, using Lewis lung cancer cells. Similar to the above, IC50 of the drugs were calculated using these measured values, and the drug concentration was obtained (Experimental Test 6-9 to Experimental Test 6-11). As a comparison, a similar test was performed for cyclophosphamide, cytarabine ocfosfate, and gefinitib (Comparative Test 6-9 to Comparative Test 6-11). IC50 determined by the cell growth inhibition test is shown in Table 11.

**[Table 11]**

| [Cell growth inhibition test result 4] Average IC50 of a hydroxyapatite-supplemented cyclophosphamide antitumor agent, hydroxyapatite-supplemented cytarabine ocfosfate antitumor agent, and hydroxyapatite-supplemented gefinitib antitumor agent determined by a Lewis lung cancer cell growth inhibition test | | | | | |
|---|---|---|---|---|---|
| Antitumor agent | Test | IC50 (ppm) | | | |
| | | -1 | -2 | -3 | -4 |
| Cyclophosphamide | Experimental Test 6-9 | 4.27 | 1.96 | 0.35 | 0.10 |
| | Comparative Test 6-9 | 18.33 | | | |
| Cytarabine ocfosfate | Experimental Test 6-10 | 12.6 | 9.8 | 7.5 | 6.2 |
| | Comparative Test 6-10 | 31.1 | | | |
| Gefinitib | Experimental Test 6-11 | 304.0 | 210.7 | 93.2 | 50.8 |
| | Comparative Test 6-11 | 414.8 | | | |

*In vitro* cell growth inhibition test was performed with the sample of Example 1-4 by methods similar to the above, using P388 lung cancer cells. Similar to the above, IC50 of the drug was calculated using these measured values, and the drug concentration was obtained (Experimental Test 6-12). As a comparison, a similar test was performed for etoposide (Comparative Test 6-12). IC50 determined by the cell growth inhibition test is shown in Table 12.

**[Table 12]**

| [Cell growth inhibition test result 5] Average IC50 of the hydroxyapatite-supplemented etoposide antitumor agent determined by a P388 lung cancer cell growth inhibition test | | | | | |
|---|---|---|---|---|---|
| Antitumor agent | Test | IC50 (ppm) | | | |
| | | -1 | -2 | -3 | -4 |
| Etoposide | Experimental Test 6-12 | 0.91 | 0.48 | 0.25 | 0.14 |
| | Comparative Test 6-12 | 2.10 | | | |

## Claims

1. An antitumor agent comprising an antitumor component blended with a hydroxyapatite, wherein the antitumor component is etoposide, interferon-β, nedaplatin, nimustine hydrochloride, carboquone, melphalan, ifosfamide, thiotepa, vinorelbine tartrate, tegafur, goserelin acetate, sobuzoxane, tretinoin, estramustine sodium phosphate, toremifene citrate, hydroxy carbamide, cytarabine ocfosfate, doxifluridine, or gefinitib, wherein hydroxyapatite has a maximum particle size of 5 µm or less, and wherein the antitumor agent is for oral administration.

2. The antitumor agent of claim 1, wherein the antitumor component is interferon-β, nedaplatin, nimustine hydrochloride, carboquone, vinorelbine tartrate, tegafur, sobuzoxane, tretinoin, estramustine sodium phosphate, toremifene citrate, hydroxy carbamide, cytarabine ocfosfate, doxifluridine, or gefinitib.

3. The antitumor agent of claim 1, wherein the antitumor component is interferon-β, nedaplatin, carboquone, vinorelbine tartrate, tegafur, or tretinoin.

4. The antitumor agent of any one of claims 1 to 3, comprising hydroxyapatite with a maximum particle size of 1 µm or less.

5. The antitumor agent of any one of claims 1 to 4, comprising hydroxyapatite with a maximum particle size of 0.5 µm or less, wherein the antitumor agent is for oral adminstration.

6. The antitumor agent of any one of claims 1 to 5, comprising hydroxyapatite with a maximum particle size of 0.1 µm or less.

7. The antitumor agent of any one of claims 1 to 6, wherein the amount of hydroxyapatite blended is 0.1 to 1000% of the antitumor component.

8. The antitumor agent of any one of claims 1 to 7, wherein the mixture of antitumor component and hydroxyapatite is pulverised.

9. An antitumor agent of any one of claims 1 to 8 for use in reducing toxicity of an antitumor component as defined in claim 1, wherein the antitumor agent is to be administered by oral administration.

## Patentansprüche

1. Antitumorwirkstoff, umfassend einen Antitumorbestandteil, der mit Hydroxylapatit vermischt ist, wobei der Antitumorbestandteil Etoposid, Interferon-β, Nedaplatin, Nimustin-Hydrochlorid, Carboquon, Melphalan, Ifosfamid, Thiotepa, Vinorelbintartrat, Tegafur, Goserelinacetat, Sobuzoxan, Tretinoin, Estramustin-Natriumphosphat, Toremifencitrat, Hydroxycarbamid, Cytarabinocfosfat, Doxifluridin, oder Gefinitib, wobei Hydroxylapatit eine maximale Partikelgröße von 5 µm oder weniger hat, und wobei der Antitumorwirkstoff zur oralen Verabreichung ist.

2. Antitumorwirkstoff nach Anspruch 1, wobei der Antitumorbestandteil Interferon-β, Nedaplatin, Nimustin-Hydrochlorid, Carboquon, Vinorelbintartrat, Tegafur, Sobuzoxan, Tretinoin, Estramustin-Natriumphosphat, Toremifencitrat, Hydroxycarbamid, Cytarabinocfosfat, Doxifluridin oder Gefinitib ist.

3. Antitumorwirkstoff nach Anspruch 1, wobei der Antitumorbestandteil Interferon-β, Nedaplatin, Carboquon, Vinorelbintartrat, Tegafur oder Tretinoin ist.

4. Antitumorwirkstoff nach einem der Ansprüche 1 bis 3, umfassend Hydroxylapatit mit einer maximalen Partikelgröße von 1 µm oder weniger.

5. Antitumorwirkstoff nach einem der Ansprüche 1 bis 4, umfassend Hydroxylapatit mit einer maximalen Partikelgröße von 0,5 µm oder weniger, wobei der Antitumorwirkstoff zur oralen Verabreichung ist.

6. Antitumorwirkstoff nach einem der Ansprüche 1 bis 5, umfassend Hydroxylapatit mit einer maximalen Partikelgröße von 0,1 µm oder weniger.

7. Antitumorwirkstoff nach einem der Ansprüche 1 bis 6, wobei die Menge des vermischten Hydroxylapatit 0,1 bis 1000% des Antitumorbestandteils ist.

8. Antitumorwirkstoff nach einem der Ansprüche 1 bis 7, wobei die Mischung aus Antitumorbestandteil und Hydroxylapatit pulverisiert ist.

9. Antitumorwirkstoff nach einem der Ansprüche 1 bis 8 zur Verwendung bei der Reduzierung der Toxizität eines wie in Anspruch 1 definierten Antitumorbestandteils, wobei der Antitumorwirkstoff oral zu verabreichen ist.

## Revendications

1. Agent antitumoral comprenant un composant antitumoral incorporé à une hydroxyapatite, dans lequel le composant antitumoral est l'étoposide, l'interféron β, le nédaplatine, le chlorhydrate de nimustine, la carboquone, le melphalan, l'ifosfamide, le thiotépa, le tartrate de vinorelbine, le tégafur, l'acétate de goséréline, le sobuzoxane, la trétinoïne, le phosphate sodique d'estramustine, le citrate de torémifène, l'hydroxycarbamide, l'ocfosfate de cytarabine, la doxifluridine, ou le géfinitib, dans lequel l'hydroxyapatite a une taille de particule maximale de 5 µm ou moins, et dans lequel l'agent antitumoral est pour une administration orale.

2. Agent antitumoral selon la revendication 1, dans lequel le composant antitumoral est l'interféron β, le nédaplatine, le chlorhydrate de nimustine, la carboquone, le tartrate de vinorelbine, le tégafur, le sobuzoxane, la trétinoïne, le phosphate sodique d'estramustine, le citrate de torémifène, l'hydroxycarbamide, l'ocfosfate de cytarabine, la doxifluridine, ou le géfinitib.

3. Agent antitumoral selon la revendication 1, dans lequel le composant antitumoral est l'interféron β, le nédaplatine, la carboquone, le tartrate de vinorelbine, le tégafur, ou la trétinoïne.

4. Agent antitumoral selon l'une quelconque des revendications 1 à 3, comprenant une hydroxyapatite ayant une taille de particule maximale de 1 µm ou moins.

5. Agent antitumoral selon l'une quelconque des revendications 1 à 4, comprenant une hydroxyapatite ayant une taille de particule maximale de 0,5 µm ou moins, dans lequel l'agent antitumoral est pour une administration orale.

6. Agent antitumoral selon l'une quelconque des revendications 1 à 5, comprenant une hydroxyapatite ayant une taille de particule maximale de 0,1 µm ou moins.

7. Agent antitumoral selon l'une quelconque des revendications 1 à 6, dans lequel la quantité d'hydroxyapatite incorporée est de 0,1 à 1000 % du composant antitumoral.

8. Agent antitumoral selon l'une quelconque des revendications 1 à 7, dans lequel le mélange du composant antitumoral et de l'hydroxyapatite est pulvérisé.

9. Agent antitumoral selon l'une quelconque des revendications 1 à 8 pour son utilisation pour réduire la toxicité d'un composant antitumoral tel que défini dans la revendication 1, dans lequel l'agent antitumoral est à administrer par administration orale.
